# EUROPEAN PATENT APPLICATION

(11) **EP 1 454 983 A1**
(43) Date of publication of application: **08.09.2004**
(21) Application number: 03028759.3
(22) Date of filing: 12.12.2003
(51) Int. Cl.: C12N 15/10

(54) **Process for recovery of nucleic acids**

(30) Priority: 07.03.2003 JP 2003060983
(71) Applicant: Hitachi High-Technologies Corporation, Tokyo 105-8717 (JP)
(72) Inventor: Sakurai, Toshinari, Hitachinaka-shi Ibaraki 312-0033 (JP); Yokobayashi, Toshiaki, Hitachinaka-shi Ibaraki 312-0032 (JP)
(74) Representative: Strehl Schübel-Hopf & Partner

(57) **Abstract**

A process for recovery of nucleic acids comprises a first step of bringing a sample containing ribonucleic acid and deoxyribonucleic acid into contact with a nucleic acid-binding solid phase; and a second step of bringing a first eluent at not lower than 4°C and not higher than 20°C into contact with said nucleic acid-binding solid phase, which has captured ribonucleic acid and deoxyribonucleic acid contained in said sample, thereby causing at least deoxyribonucleic acid to be eluted into the first eluent.

## Description

### BACKGROUND OF THE INVENTION

The present invention relates to a process for recovery of nucleic acids from a sample containing ribonucleic acid and deoxyribonucleic acid. More particularly, the present invention relates to a process for selectively recovering deoxyribonucleic acid (DNA) and/or ribonucleic acid (RNA).

Japanese Patent No. 2680462 discloses that nucleic acid can be extracted with the help of silica particles, as the solid phase for nucleic acid binding, capable of binding to nucleic acid in the presence of a chaotropic agent. According to this disclosure, extraction is achieved in the following way. First, a sample containing nucleic acids is placed in a reaction vessel containing a suspension of silica particles and a buffer solution of guanidine thiocyanate as a chaotropic agent. After stirring, the reaction product is centrifuged to precipitate the complex composed of silica particles and nucleic acids binding thereto. With the supernatant discarded, the remaining complex is washed with a washing liquid and then reprecipitated. The resulting complex is washed with an aqueous ethanol solution and then with acetone. With acetone removed by drying, the complex undergoes elution with a buffer solution for recovery of nucleic acids.

Also, Japanese Patent Laid-open No. 11-266864 discloses a process and apparatus for purifying nucleic acids with the help of a nucleic acid-capturing tip holding a silica-containing solid phase therein. According to this disclosure, purification is accomplished in the following manner. First, a nucleic acid-capturing tip is detachably connected to a valved nozzle for admission and emission of fluid. The tip is charged, by aspiration from a container, with a mixed solution of a nucleic acid-containing sample and a substance to promote the binding of nucleic acid to the solid phase. With nucleic acid binding to the solid phase, the tip is cleared of liquid and then washed with a washing liquid which is aspirated and discharged. The tip is charged with an eluate by aspiration, and the eluate which contains nucleic acid released from the solid phase is discharged into a container to receive the purified product.

In addition, Japanese Patent Laid-open No. 9-327291 discloses a process for extracting and purifying RNA with the help of a nucleic acid-binding carrier. According to this process, a biological material such as cells is added to, mixed with, or brought into contact with a solution containing a chaotropic agent, an extractant of organic solvent, and a nucleic acid-binding carrier under an acidic condition, so that RNA contained in the biological material is adsorbed onto the carrier as a solid phase. The carrier is washed with a washing solution and finally RNA is eluted with an eluate.

Furthermore, Japanese Patent Laid-open No. 11-196869 discloses a process for isolating ribonucleic acid with the help of a nucleic acid-binding carrier. According to this process, a sample containing RNA is mixed with an acidic solution containing a chaotropic agent, a watersoluble organic solvent, and a nucleic acid-binding carrier, so that RNA is adsorbed onto the carrier. The resulting carrier-RNA complex is separated from the liquid phase, and washed if necessary, and finally RNA is eluted from the carrier-RNA complex.

The processes disclosed in Japanese Patent No. 2680462 and Japanese Patent Laid-open No. 11-266864 cause DNA and RNA to be recovered in the form of mixture. Therefore, they need an additional step for separation or enzyme treatment to isolate DNA from the mixture.

Also, the processes disclosed in Japanese Patent Laid-open Nos. 9-327291 and 11-196869 cannot isolate RNA and DNA from the same sample because DNA is removed as an unadsorbed component in the series of steps.

All the processes disclosed in the patent documents noted above need special treatment for removal of RNA if a purified RNA solution free of RNA is to be obtained from the same sample. In other words, they need subdivision of the same sample into small portions and separate steps for recovery of DNA and RNA with the help of a plurality of protocols. Thus, the conventional technologies need very complicated and inefficient treatment for isolation and purification of DNA and RNA from the same sample.

### SUMMARY OF THE INVENTION

It is an object of the present invention to provide a process for recovering nucleic acids. This process permits deoxyribonucleic acid and/or ribonucleic acid to be recovered efficiently from a sample containing both deoxyribonucleic acid and ribonucleic acid.

The present invention is directed to a process for recovery of nucleic acids, which involves bringing an eluent at a low temperature into contact with a nucleic acid-capturing substance which has captured ribonucleic acid (RNA) and deoxyribonucleic acid (DNA). The rate of recovery of RNA and DNA by elution varies depending on the temperature of the eluate. With an eluent at a low temperature, the rate of recovery of DNA is much higher than that of RNA. This permits DNA to be recovered efficiently from a sample containing both RNA and DNA.

The present invention is directed also to a process for recovery of nucleic acids. According to this process, a nucleic acid-capturing substance that has captured RNA and DNA is treated with a first eluent and subsequently with a second eluent at a higher temperature than the first one. The first eluent elutes most of DNA from the nucleic acid-capturing substance, with most of RNA remaining on the nucleic acid-capturing substance. Then, the second eluent elutes most of RNA from the nucleic acid-capturing substance. In this way it is possible to selectively recover DNA and RNA from one sample.

Furthermore, the present invention is directed to a process for recovery of nucleic acids, which involves the addition of a reagent that decreases the amount of RNA in the first eluent in which most of DNA has been eluted. Since the first eluate merely contains a very small amount of RNA, the reagent to reduce RNA permits the recovery of high-purity DNA even though it is used in small amounts.

In addition, the present invention is directed to a process for recovery of nucleic acids, which involves the addition of a reagent that decreases the amount of DNA in the second eluent in which most of RNA has been eluted. Since the second eluate merely contains a very small amount of DNA, the reagent to reduce DNA permits the recovery of high-purity RNA even though it is used in small amounts.

These and other objects, advantages, and features of the invention well be readily ascertained by referring to the following description and appended drawings.

### BRIEF DESCRIPTION OF THE DRAWINGS

Other objects and advantages of the invention will become apparent from the following description of embodiments with reference to the accompanying drawings in which:
Fig. 1 is a flow chart illustrating the process for recovery of nucleic acids according to the present invention;
Fig. 2 is a sectional view of the nucleic acid-capturing tip;
Fig. 3 is a schematic diagram illustrating the apparatus for eluting nucleic acids captured by the nucleic acid-capturing solid phase; and
Fig. 4 is a diagram showing the relationships between the rate of elution of DNA and the temperature of elution, and the rate of elution of RNA and the temperature of elution.

### DETAILED DESCRIPTION OF THE PREFERRED EMBODIMENTS

According to the present invention, the process for recovery of nucleic acids consists of a first step of bringing a sample containing ribonucleic acid and deoxyribonucleic acid into contact with a nucleic acid-binding solid phase and a second step of bringing a first eluate at a first temperature into contact with the nucleic acid-binding solid phase, which has captured ribonucleic acid and deoxyribonucleic acid contained in the sample, with the first temperature ranging from a temperature at which deoxyribonucleic acid is effectively eluted from the nucleic acid-binding solid phase to a temperature at which ribonucleic acid is effectively eluted from the nucleic acid-binding solid phase.

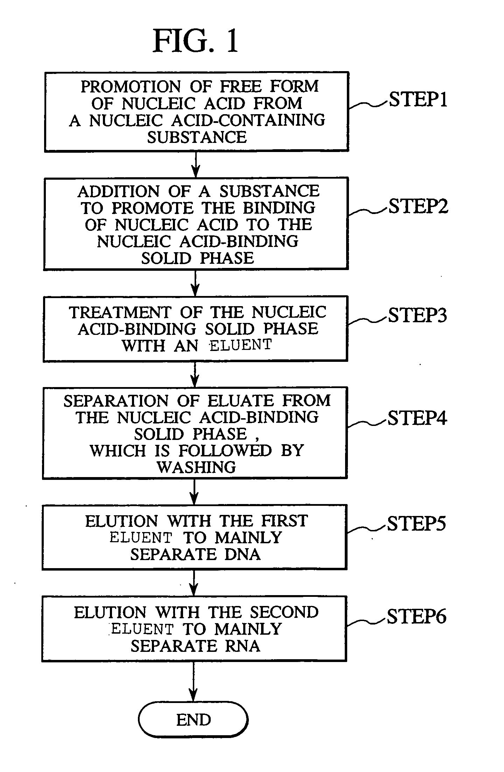

The process for recovery of nucleic acids proceeds according to the flow chart shown in Fig. 1. As Fig. 1 shows, the process for recovery of nucleic acids starts with Step 1 which is intended to promote the free form of nucleic acid from a nucleic acid-containing substance. By the term "nucleic acid-containing substance" is meant a sample containing deoxyribonucleic acid (DNA for short hereinafter) and ribonucleic acid (RNA for short hereinafter). It includes, for example, organic samples such as whole blood, serum, sputum, and urine, biological samples such as cultured cells and bacteria, gel holding nucleic acid left after electrophoresis, reaction products of DNA amplifying enzyme, and any substance containing crude nucleic acid. Incidentally, "nucleic acid" embraces DNA and RNA of two-chain structure or one-chain structure throughout or partially.

Free form of nucleic acid from a nucleic acid-containing substance can be promoted if the nucleic acid-containing substance is made into an adequate solution in which nucleic acid is readily released from the nucleic acid-containing substance. This object is achieved by adding a reagent that promotes free form of nucleic acid to the solution containing a nucleic acid-containing substance. Such a reagent is not specifically restricted unless it is excessively viscous. It includes, for example, proteolytic enzyme such as proteinase K, surface-active agent such as sodium dodecylsulfate, and chaotropic agent such as guanidinium salt. In the case where the nucleic acid to be recovered is RNA, it is desirable to add ribonuclease inhibitor or guanidine thiocyanate to the solution containing a nucleic acid-containing substance so as to prevent the actions of ribonuclease.

In Step 2, the solution prepared in Step 1 is given a reagent which causes the nucleic acid which has been released into the solution in Step 1 to bind to the nucleic acid-binding solid phase with which the solution is brought into contact in the subsequent step. The reagent used in Step 2 is not specifically restricted; it should preferably be a chaotropic agent, e.g., guanidine-HCl, in the case where the nucleic acid-binding solid phase contains silica. In addition, it is desirable to add guanidine thiocyanate in Step 2 for the same reason as in Step 1, in the case where the nucleic acid to be recovered is RNA. Guanidine thiocyanate should preferably be added such that its final concentration is not less than 3 mol/L and the final pH is low. Alternatively, it is also possible to carry out Step 1 and Step 2 together, as described in Japanese patent No. 2680462.

Step 3 is intended to bring the solution prepared in Step 2 into contact with a nucleic acid-binding solid phase. The nucleic acid-binding solid phase is not specifically restricted so long as it is capable of fixing nucleic acid onto the surface thereof. It includes, for example, any substance containing silicon oxide, such as glass particles, silica particles, silica filter and crushed product thereof, silica wool, and diatomaceous earth, and silica-coated plastic materials. Of these examples, those that contain silica or silicon dioxide are preferable.

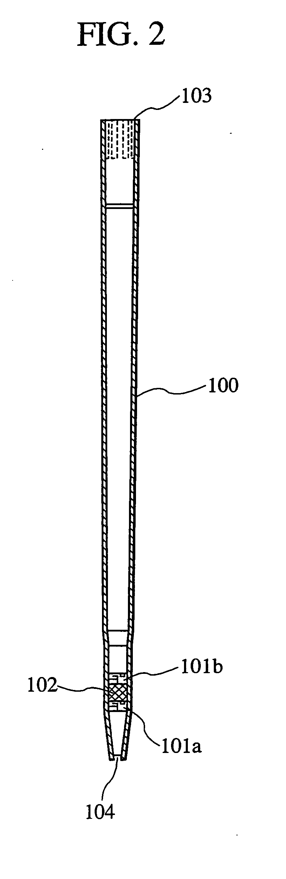

In the case where the nucleic acid-binding solid phase is in the form of particles or granules, it is possible to bring the solution prepared in Step 2 into contact with the nucleic acid-binding solid phase by mixing with stirring the particles or granules with the solution prepared in Step 2. On the other hand, in the case where the nucleic acid-binding solid phase is placed in the nucleic acid-capturing tip 100 as shown in Fig. 2, it is possible to bring the solution prepared in Step 2 into contact with the nucleic acid-binding solid phase in Step 3 by causing the solution prepared in Step 2 to be repeatedly aspirated in and discharged from the nucleic acid-capturing tip 100.

To be concrete, the nucleic acid-capturing tip 100 is a thin tubular object as shown in Fig. 2. It has a head 103 in which the nozzle of a pipet (not shown) tightly fits. It is gradually tapered to its lower end 104. It holds the nucleic acid-binding solid phase 102 near its lower end. The nucleic acid-capturing tip 100 has a lower discoid stopping member 101a which is press fitted therein and an upper discoid stopping member 101b. The lower discoid stopping member 101a prevents the nucleic acid-binding solid phase 102 from flowing out and defines the average distance of the nucleic acid-binding solid phase 102. The upper discoid stopping member 101b is positioned so that the nucleic acid-binding solid phase 102 has a prescribed average distance. These discoid stopping members 101a and 101b have a large number of pores that permit easy passage of liquid and gas. These pores are small enough to prevent the nucleic acid-binding solid phase 102 from flowing out.

The nucleic acid-capturing tip 100 is made of transparent or translucent synthetic resin. The discoid stopping members 101a and 101b are formed from silica particles, 0.1 mm in diameter, by sintering, so that they fit in the nucleic acid-capturing tip 100 at prescribed positions. The nucleic acid-binding solid phase 102 is, for example, silica wool (grade B, from Toshiba Chemical).

In Step 4, the nucleic acid-binding solid phase is separated from the solution and the nucleic acid-binding solid phase is washed. In the case of a nucleic acid-binding solid phase in particulate or granular form, separation of the nucleic acid-binding solid phase from the solution may be accomplished by mixing and stirring in Step 3 and then removing the solution only by aspiration or the like. In the case where a nucleic acid-capturing solid phase placed in the nucleic acid-capturing tip 100 is used, separation of the nucleic acid-binding solid phase from the solution may be accomplished by discharging the solution remaining in the nucleic acid-capturing tip 100.

In Step 4, the nucleic acid-binding solid phase is washed after separation of the nucleic acid-binding solid phase from the solution. Washing of the nucleic acid-binding solid phase is accomplished by treating the nucleic acid-binding solid phase with a washing agent. The washing solution is not specifically restricted; it should preferably be one, for example, which can remove the reagents added in Steps 1 and 2 from the nucleic acid-binding solid phase, while allowing nucleic acid to keep binding to the nucleic acid-binding solid phase. Examples of the washing agent include ethyl alcohol and isopropyl alcohol in concentration higher than 70%. In the case where the nucleic acid recovered by the process mentioned herein is used for PCR and the like it is desirable that the washing agent contains ethyl alcohol or isopropyl alcohol in as low concentrations as possible so long as it produces its washing effect. In this way it is possible to prevent the ethyl alcohol or isopropyl alcohol contained in the washing agent from adversely affecting PCR. A preferred example of the washing agent is 50% ethyl alcohol containing 25 mmol/L potassium acetate. This washing agent prevents the component therein from producing an adverse effect in the course of PCR.

In Step 5, nucleic acid is eluted from the nucleic acid-binding solid phase that has been washed in Step 4. In Step 5, the nucleic acid-binding solid phase that has been washed in Step 4 is treated with the first eluate at a temperature within a certain range (referred to as the first temperature range hereinafter). Above this temperature, DNA is effectively eluted from the nucleic acid-binding solid phase; below this temperature, RNA is effectively eluted from the nucleic acid-binding solid phase. The first eluate is not specifically restricted and any known eluate may be used so long as it is capable of eluting DNA from the nucleic acid-binding solid phase. An example of the first eluate is pure water or a dilute aqueous solution, such as the one containing 10 mmol/L tris (pH 8.5) and 0.1 mmol/L EDTA.

In Step 5, the nucleic acid-binding solid phase is treated with the first eluate at a temperature in the first temperature range. This treatment is intended to elute only DNA from the nucleic acid which has bound to the nucleic acid-binding solid phase. Treatment in this manner in Step 5 permits most RNA binding to the nucleic acid-binding solid phase to keep binding to the nucleic acid-binding solid phase. Therefore, the first eluate that has undergone Step 5 mostly contains DNA out of the nucleic acid binding to the nucleic acid-binding solid phase. The thus obtained solution that mostly contains DNA may be used as such in a subsequent step; however, it may be converted into a high-purity DNA solution by incorporation with a ribonuclease not containing deoxyribonuclease. The ribonuclease used in this step should preferably be ribonuclease A. The amount of ribonuclease may be reduced because DNA is predominant.

The present inventors found that how DNA or RNA binds to the nucleic acid-binding solid phase is governed by a special rule. The finding is that DNA is eluted from the nucleic acid-binding solid phase at a much lower temperature than RNA is eluted from the nucleic acid-binding solid phase. This is the reason why DNA can be selectively eluted by treatment at a temperature within the first temperature range.

The first temperature range extends from a temperature at which DNA is effectively eluted from the nucleic acid-binding solid phase to a temperature at which RNA is effectively eluted from the nucleic acid-binding solid phase. Thus, the first temperature range is defined as a series of temperatures at which not more than 10% of RNA is eluted from the nucleic acid-binding solid phase. To be concrete, the first temperature range should extend from 4°C to 20°C, preferably from 15°C to 20°C.

The first temperature range may be defined in another way as a series of temperatures at which the elution (with a standard eluate containing tris and EDTA) of nucleic acid from the nucleic acid-binding solid phase carrying DNA and RNA in equal amounts takes place such that the content of RNA in the eluate is not more than 50% of the content of DNA in the eluate.

In the case where the nucleic acid to be recovered is DNA, the procedure consisting of Steps 1 to 5 mentioned above gives a DNA-rich solution containing very little RNA. However, an additional Step 6 is necessary if DNA and RNA are to be selectively recovered or RNA alone or free of DNA is to be recovered.

Step 6 consists of adding a second eluate to the nucleic acid-binding solid phase from which most DNA has been eluted in Step 5. Elution is carried out within a range of temperatures (referred to as the second temperature range hereinafter) at which RNA is effectively eluted from the nucleic acid-binding solid phase. Incidentally, Step 6 may or may not be preceded by washing the nucleic acid-binding solid phase that has undergone Step 5 in the same way as in Step 4.

The second eluate is not specifically restricted so long as it is capable of eluting RNA from the nucleic acid-binding solid phase; any known one can be used as in the case of the first eluate. Also, the second eluate may have the same composition as the first one. The second eluate may be pure water or a dilute aqueous solution. A preferred example of the second eluate is a solution containing 10 mmol/L Bicine (pH 8.5) and 0.1 mmol/L EDTA.

The second temperature range is defined as a series of temperatures, not lower than 40°C, at which not less than 70% of RNA is eluted from the nucleic acid-binding solid phase. To be concrete, the second temperature range extends from 40°C to 70°C. In Step 6, treatment at a temperature not higher than 80°C prevents RNA and the nucleic acid-binding solid phase from decomposition.

In Step 6, treatment of the nucleic acid-binding solid phase with the second eluate at a temperature within the second temperature range elutes RNA from the nucleic acid-binding solid phase. This treatment in Step 6 also elutes DNA remaining uneluted in Step 5 from the nucleic acid-binding solid phase. However, the solution obtained by treatment in Step 6 contains mostly RNA if DNA has been thoroughly eluted in Step 5. The thus obtained RNA-rich solution may be used as such for treatment in the subsequent step. However, it may be incorporated with DNase free of RNase to give a high-purity RNA solution. The DNase should preferably be deoxyribonuclease I. Incidentally, the amount of DNase may be reduced because the solution contains mostly RNA.

As mentioned above, the procedure in Steps 5 and 6 makes it possible to selectively recover DNA and RNA from the same sample containing nucleic acid. In other words, there is no need for dividing one nucleic acid-containing sample into two, one for recovery of DNA and the other for recovery of RNA. This permits DNA and RNA to be selectively recovered with certainty from a very small amount of nucleic acid-containing substance used in Step 1.

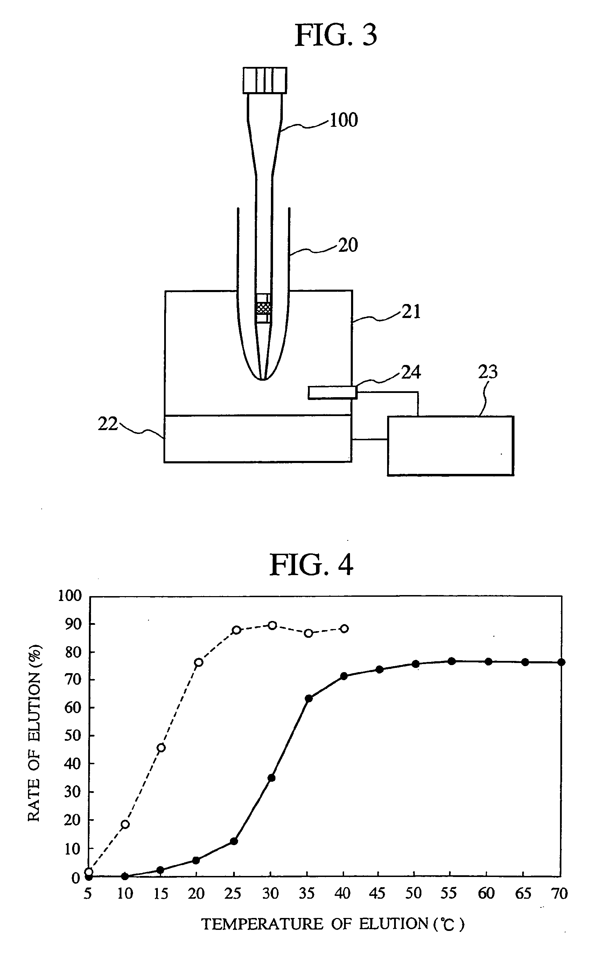

Incidentally, the treatment in Steps 5 and 6 may be accomplished in a temperature-controlled container 20 as shown in Fig. 3. The container 20 is placed in a hollow formed in a temperature-controlled block 21. It holds a solution and the nucleic acid-capturing tip 100 shown in Fig. 1. The solution in the container 20 is kept at a desired temperature by the temperature-controlled block 21.

Incidentally, the container 20 may be formed from any material so long as it can hold a liquid therein; however, it should preferably be formed from a material having high thermal conductivity. Moreover, it should preferably be formed thin so that it effectively transmits heat from the block 21 to the liquid held therein.

In addition, the temperature-controlled block 21 has a heat source 22 such as a Peltier element in contact therewith. Therefore, it is adjusted to a desired temperature by the heat source 22, which is kept at a desired temperature by a controller 23. A temperature sensor 24 is attached to the temperature-controlled block 21. The controller 23 works on receiving feedback from the sensor 24 which measures the temperature of the temperature-controlled block 21.

Incidentally, the temperature-controlled block 21 may be formed from any material, such as metal, having high thermal conductivity. It may also be formed from an amorphous material capable of temperature control by the heat source 22. The heat source 22 may be one which only has a heating function if it is not necessary to keep the liquid in the container below room temperature.

As mentioned above, the procedure in Steps 5 and 6 may be carried out under strict temperature control, with the first or second eluate placed in the temperature-controlled container 20 shown in Fig. 3. In this way it is possible to selectively recover DNA and RNA.

### [Examples]

The invention will be described in more detail with reference to the following examples, which are not intended to restrict the scope thereof.

### [Example 1]

This example demonstrates recovery of DNA and RNA by the process which consists of capturing DNA and RNA by using the nucleic acid-capturing tip 100 shown in Fig. 2 and performing elution at different temperatures.

The sample used for recovery of DNA was serum taken from a patient with hepatitis B. To the serum sample (200 µL) was added a first reagent (700 µL), which is a buffer solution of MES (2-morpholino-ethanesulfonic acid, made by Dojin Laboratories) containing 8% Triton X-100 (made by LKB) and 5.5 mol/L of guanidine thiocyanate (biochemical grade, made by Wako Pure Chemical Industries, Ltd.) After stirring, the mixed solution was allowed to stand for 10 minutes at room temperature so that virus was dissolved.

After standing for 10 minutes, the mixed solution was given a second reagent (100 µL), which is a buffer solution of MES (made by Dojin Laboratories) containing 1% Triton X-100 (made by LKB) and 5.0 mol/L of guanidine thiocyanate (biochemical grade, made by Wako Pure Chemical Industries, Ltd.). After stirring, the mixed solution was aspirated in and discharged from the nucleic acid-capturing tip 100, shown in Fig. 2, which is attached to a syringe. This operation is repeated 20 times in such a way that the mixed solution does not pass through the stopping member 101a at the time of aspiration and does not pass through the stopping member 101b at the time of discharging.

After 20 repeated operations of aspiration and discharging, the mixed solution was discharged from the nucleic acid-capturing tip by means of the syringe in such a way that the level of the mixed solution did not pass through the stopping member 101b. Then, the second reagent (900 µL) was aspirated in and discharged from the nucleic acid-capturing tip by means of the syringe. This operation was repeated 5 times in such a way that the mixed solution did not pass through the stopping member 101a at the time of aspiration and did not pass through the stopping member 101b at the time of discharging. After the second reagent was discharged, the third reagent of 900 µL was aspirated in and discharged from the nucleic acid-capturing tip by means of the syringe. This operation was repeated 5 times. The third reagent was 50% ethanol (reagent grade, made by Wako Pure Chemical Industries, Ltd.) containing 25 mmol/L potassium acetate (reagent grade, made by Wako Pure Chemical Industries, Ltd.)

After five repeated operations of aspiration and discharging, the mixed solution was entirely discharged from the nucleic acid-capturing tip 100 by means of the syringe. Further, the mixing solution remaining in the nucleic acid-capturing tip 100 was removed by blowing. Then, washing with the third reagent was repeated. The residual third reagent was removed by blowing. Then, the nucleic acid-capturing tip 100 was caused, by means of the syringe, to repeatedly, 20 times, aspirate and discharge 60 µL of a first eluate which is kept at a prescribed temperature by the block incubator (shown in Fig. 3). The first eluate was a 10 mmol/L Tris buffer solution (pH 8.5) (made by Dojin Laboratories) containing 0.1 mmol/L ethylenediamine tetraacetic acid (genetic engineering grade, made by Nippon Gene Co., Ltd.) After the repeated aspiration and discharging operations (20 times), the concentration of DNA contained in the first eluate was determined to calculate the rate of DNA elution.

On the other hand, recovery of RNA was carried out by using a serum sample taken from a patient with hepatitis C. The procedure for recovery of the gene of virus of hepatitis B was repeated. Incidentally, the procedure for recovery of RNA employed the second eluate in place of the first eluate. The second eluate was a 10 mmol/L Bicine buffer solution (pH 8.5) (made by Dojin Laboratories) containing 0.1 mmol/L ethylenediamine tetraacetic acid (genetic engineering grade, made by Nippon Gene Co., Ltd.) Then, the concentration of RNA contained in the second eluate was determined to calculate the rate of RNA elution.

In Fig. 4, the broken line and the solid line show, respectively, the relation between the temperature of the first eluate and the rate of elution of DNA and the relation between the temperature of the second eluate and the rate of elution of RNA.

It is apparent from Fig. 4 that elution of DNA and RNA from the nucleic acid-binding solid phase takes place respectively at temperatures in different ranges. Elution of DNA starts at a comparatively low temperature, whereas elution of RNA starts at a comparatively high temperature. The foregoing indicates that DNA can be collected in the first eluate if the nucleic acid-binding solid phase with nucleic acid binding thereto is treated with the first eluate at a temperature which is higher than the temperature for effective elution of DNA from the nucleic acid-binding solid phase and which is lower than the temperature for effective elution of RNA from the nucleic acid-binding solid phase. The foregoing also indicates that RNA can be collected in the second eluate if the nucleic acid-binding solid phase from which DNA has been recovered is treated with the second eluate at a temperature which is higher than the temperature for elution of RNA from the nucleic acid-binding solid phase.

It is apparent from these results that it is possible to selectively recover DNA and RNA by using the eluate at controlled temperatures even in the case where the nucleic acid-capturing solid phase has captured both DNA and RNA from a sample containing both DNA and RNA.

It is particularly apparent from Fig. 4 that it is possible to recover mainly DNA if the nucleic acid-binding solid phase that has captured DNA and RNA is treated with an eluate at a temperature not lower than 4°C and not higher than 20°C. It is also apparent from Fig. 4 that it is possible to recover RNA from the nucleic acid-binding solid phase with RNA binding thereto if the nucleic acid-binding solid phase from which DNA has been recovered is treated with an eluate at a temperature not lower than 40°C.

According to the present invention, it is possible to efficiently recover deoxyribonucleic acid and/or ribonucleic acid from a sample containing deoxyribonucleic acid and ribonucleic acid.

While the invention has been described in its preferred embodiments, it is to be understood that the words which have been used are words of description rather than limitation and that changes within the purview of the appended claims may be made without departing from the true scope and spirit of the invention in its broader aspects.

## Claims

1. A process for recovery of nucleic acids, comprising:
bringing a sample containing ribonucleic acid and deoxyribonucleic acid into contact with a nucleic acid-binding solid phase; and
bringing a first eluent, at not lower than 4°C and not higher than 20°C into contact with said nucleic acid-binding solid phase, which has captured ribonucleic acid and deoxyribonucleic acid contained in said sample, thereby causing at least deoxyribonucleic acid to be eluted into the first eluate.

2. The process for recovery of nucleic acids as defined in Claim 1, further comprising bringing a second eluent at a higher temperature than the first eluent into contact with the nucleic acid-binding solid phase after said second step, thereby causing at least ribonucleic acid to be eluted into the second eluate.

3. The process for recovery of nucleic acids as defined in Claim 1, wherein the first eluent into which at least deoxyribonucleic acid has been eluted is incorporated with a reagent to reduce the amount of ribonucleic acid.

4. The process for recovery of nucleic acids as defined in Claim 2, wherein the second eluent into which at least ribonucleic acid has been eluted is incorporated with a reagent to reduce the amount of deoxyribonucleic acid.

5. The process for recovery of nucleic acids as defined in Claim 1, wherein the nucleic acid-binding solid phase is a silica-containing substance.

6. A process for recovery of nucleic acids, comprising:
bringing a sample containing ribonucleic acid and deoxyribonucleic acid into contact with a nucleic acid-binding solid phase;
bringing a first eluent into contact with said nucleic acid-binding solid phase, which has captured ribonucleic acid and deoxyribonucleic acid contained in said sample, thereby causing at least deoxyribonucleic acid to be eluted into the first eluent; and
bringing a second eluent at a higher temperature than the first eluent into contact with the nucleic acid-binding solid phase, with which the first eluent has been brought into contact, thereby causing at least ribonucleic acid to be eluted into the second eluent.

7. The process for recovery of nucleic acids as defined in Claim 6, wherein the first eluent is at a temperature not lower than 4°C and not higher than 20°C.

8. The process for recovery of nucleic acids as defined in Claim 6, wherein the first eluent into which at least deoxyribonucleic acid has been eluted is incorporated with a reagent to reduce the amount of ribonucleic acid.

9. The process for recovery of nucleic acids as defined in Claim 6, wherein the first eluent into which at least ribonucleic acid has been eluted is incorporated with a reagent to reduce the amount of deoxyribonucleic acid.

10. The process for recovery of nucleic acids as defined in Claim 6, wherein the nucleic acid-binding solid phase is a silica-containing substance.
